# DEMANDE DE BREVET EUROPEEN

(11) **EP 1 676 839 A2**
(43) Date de publication de la demande: **05.07.2006**
(21) Numéro de dépôt: 06075789.5
(22) Date de dépôt: 06.07.2001
(51) Int. Cl.: C07D 209/42, A61K 31/404, A61P 9/00

(54) **Nouvelle forme cristalline bêta du sel de tert-butylamine du perindopril, son procédé de préparation et les compositions pharmaceutiques qui la contiennent**

(30) Priorité: 06.07.2000 FR 0008792
(62) Demande divisionnaire de: 01954059.0
(71) Demandeur: Les Laboratoires Servier, 92415 Courbevoie Cedex (FR)
(72) Inventeur: Pfeiffer, Bruno, 95320 Saint Leu La Foret (FR); Ginot, Yves-Michel, 45000 Orleans (FR); Coquerel, Gérard, 76520 Boos (FR); Beilles, Stéphane, 21800 Neuilly Les Dijon (FR)

(57) **Abrégé**

Forme cristalline β du composé de formule (I) : 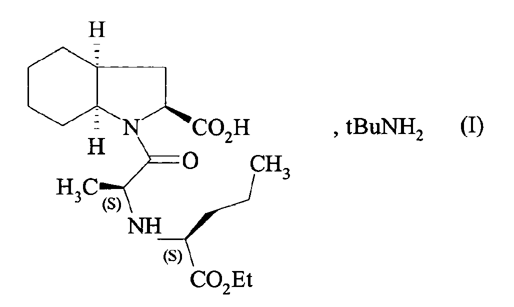 caractérisée par son diagramme de diffraction X sur poudre. Médicaments.

## Description

La présente invention concerne une nouvelle forme cristalline β du sel de tert-butylamine du perindopril de formule (I) : 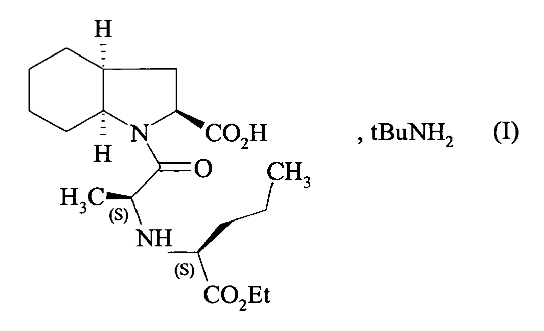 son procédé de préparation ainsi que les compositions pharmaceutiques qui la contiennent.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine 1 (ou kininase II), ce qui permet d'une part d'empêcher la transformation du décapeptide angiotensine 1 en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière, d'humidité ou de taux d'oxygène.

Le brevet EP 0 308 341 décrit un procédé de synthèse industrielle du perindopril. Cependant, ce document ne précise pas les conditions d'obtention du perindopril sous une forme présentant ces caractéristiques de manière reproductible.

La demanderesse a présentement trouvé qu'un sel particulier du perindopril, le sel de tert-butylamine, pouvait être obtenu sous une forme cristalline bien définie, parfaitement reproductible et présentant notamment des caractéristiques intéressantes de formulation.

Plus spécifiquement, la présente invention concerne la forme cristalline β du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, d'intensité et d'intensité relative (exprimée en pourcentage par rapport à la raie la plus intense) :

| **Angle 2 thêta (°)** | **Distance inter-réticulaire d (Å)** | **Intensité** | **Intensité relative (%)** |
|---|---|---|---|
| 5,169 | 17,08 | 523 | 16,5 |
| 8,379 | 10,54 | 1001 | 31,5 |
| 9,350 | 9,45 | 3175 | 100 |
| 14,746 | 6,00 | 236 | 7,4 |
| 15,411 | 5,74 | 753 | 23,7 |
| 15,931 | 5,56 | 279 | 8,8 |
| 16,711 | 5,30 | 113 | 3,6 |
| 18,161 | 4,88 | 122 | 3,8 |
| 20,564 | 4,32 | 1198 | 37,7 |
| 21,285 | 4,17 | 330 | 10,4 |
| 21,781 | 4,08 | 317 | 10 |
| 22,632 | 3,93 | 190 | 6 |
| 23,308 | 3,81 | 133 | 4,2 |
| 23,797 | 3,74 | 427 | 13,4 |
| 24,276 | 3,66 | 118 | 3,7 |
| 25,190 | 3,53 | 92 | 2,9 |
| 25,924 | 3,43 | 251 | 7,9 |
| 26,646 | 3,34 | 250 | 7,9 |
| 27,620 | 3,23 | 96 | 3 |
| 28,306 | 3,15 | 133 | 4,2 |

L'invention s'étend également au procédé de préparation de la forme cristalline β du composé de formule (I), caractérisé en ce que :
- soit, selon une première variante, on porte à reflux une solution du sel de tert-butylamine du perindopril dans le dichlorométhane, puis on refroidit rapidement la solution à 0°C et collecte le solide obtenu par filtration,
- soit, selon une seconde variante, on porte à reflux une solution du sel de tert-butylamine du perindopril dans l'acétate d'éthyle, on refroidit rapidement la solution à 5°C, puis on collecte le solide obtenu par filtration.
   - Dans le procédé de cristallisation selon l'invention, on peut utiliser le composé de formule (I) obtenu par n'importe quel procédé. Avantageusement, on utilise le composé de formule (I) obtenu par le procédé de préparation décrit dans le brevet EP 0 308 341.
   - Dans la première variante du procédé selon l'invention, la concentration du composé de formule (I) dans le dichlorométhane est préférentiellement comprise entre 100 et 200 g/l.
   - Dans la seconde variante du procédé selon l'invention, la concentration du composé de formule (I) dans l'acétate d'éthyle est préférentiellement comprise entre 70 et 90 g/l.

L'invention s'étend aussi aux compositions pharmaceutiques renfermant comme principe actif la forme cristalline β du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les compositions pharmaceutiques selon l'invention peuvent également contenir un diurétique comme l'indapamide.

Les exemples suivants illustrent l'invention mais ne la limitent en aucune façon.

Le spectre de diffraction X sur poudre a été mesuré avec les conditions expérimentales suivantes :
- Diffractomètre Siemens D5005, détecteur à scintillations,
- Anticathode de cuivre (λ=1,5405 Å), voltage 40 KV, intensité 40mA,
- Montage θ-θ,
- Domaine de mesures : 5° à 30°,
- Incrémentation entre chaque mesure : 0,02°,
- Temps de mesure par pas : 2s,
- Fentes variables : v6,
- Filtre Kβ (Ni),
- Pas de référence interne,
- Procédure de zéro avec les fentes Siemens,
- Données expérimentales traitées avec le logiciel EVA (version 5.0).

### EXEMPLE 1 : Forme cristalline β du sel de tert-butylamine du perindopril

135 g du sel de tert-butylamine du perindopril obtenu selon le procédé décrit dans le brevet EP 0 308 341 sont dissous dans 1100 ml de dichlorométhane portés au reflux. La solution est ensuite refroidie à 0°C et le solide obtenu est collecté par filtration.

### Diagramme de diffraction X sur poudre :

Le profil de diffraction des rayons X de la poudre (angles de diffraction) de la forme β du sel de tert-butylamine du perindopril est donné par les raies significatives rassemblées dans le tableau suivant, avec l'intensité et l'intensité relative (exprimée en pourcentage par rapport à la raie la plus intense) :

| **Angle 2 thêta (°)** | **Distance inter-réticulaire d (Å)** | **Intensité** | **Intensité relative (%)** |
|---|---|---|---|
| 5,169 | 17,08 | 523 | 16,5 |
| 8,379 | 10,54 | 1001 | 31,5 |
| 9,350 | 9,45 | 3175 | 100 |
| 14,746 | 6,00 | 236 | 7,4 |
| 15,411 | 5,74 | 753 | 23,7 |
| 15,931 | 5,56 | 279 | 8,8 |
| 16,711 | 5,30 | 113 | 3,6 |
| 18,161 | 4,88 | 122 | 3,8 |
| 20,564 | 4,32 | 1198 | 37,7 |
| 21,285 | 4,17 | 330 | 10,4 |
| 21,781 | 4,08 | 317 | 10 |
| 22,632 | 3,93 | 190 | 6 |
| 23,308 | 3,81 | 133 | 4,2 |
| 23,797 | 3,74 | 427 | 13,4 |
| 24,276 | 3,66 | 118 | 3,7 |
| 25,190 | 3,53 | 92 | 2,9 |
| 25,924 | 3,43 | 251 | 7,9 |
| 26,646 | 3,34 | 250 | 7,9 |
| 27,620 | 3,23 | 96 | 3 |
| 28,306 | 3,15 | 133 | 4,2 |

### EXEMPLE 2 : Forme cristalline β du sel de tert-butylamine du perindopril

125 g du sel de tert-butylamine du perindopril obtenu selon le procédé décrit dans le brevet EP 0 308 341 sont dissous dans 1,5 1 d'acétate d'éthyle portés au reflux.
La solution est ensuite refroidie rapidement à 5°C et le solide obtenu est collecté par filtration.

### EXEMPLE 3 : Composition pharmaceutique

Formule de préparation pour 1000 comprimés dosés à 4 mg :

| | |
|---|---|
| Composé de l'exemple 1 | 4 g |
| Hydroxypropylcellulose | 2 g |
| Amidon de blé | 10 g |
| Lactose | 100 g |
| Stéarate de magnésium | 3 g |
| Talc | 3 g |

## Revendications

1. Forme cristalline β du composé de formule (I) : 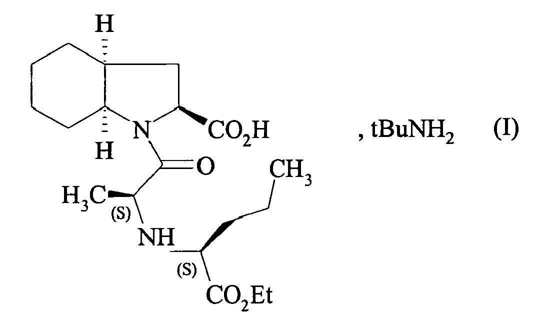 **caractérisée par** le diagramme de diffraction X sur poudre suivant, mesuré sur un diffractomètre (anticathode de cuivre) et exprimé en termes de distance inter-réticulaire d, d'angle de Bragg 2 thêta, d'intensité et d'intensité relative (exprimée en pourcentage par rapport à la raie la plus intense :
| **Angle 2 thêta (°)** | **Distance inter-réticulaire d (Å)** | **Intensité** | **Intensité relative (%)** |
|---|---|---|---|
| 5,169 | 17,08 | 523 | 16,5 |
| 8,379 | 10,54 | 1001 | 31,5 |
| 9,350 | 9,45 | 3175 | 100 |
| 14,746 | 6,00 | 236 | 7,4 |
| 15,411 | 5,74 | 753 | 23,7 |
| 15,931 | 5,56 | 279 | 8,8 |
| 16,711 | 5,30 | 113 | 3,6 |
| 18,161 | 4,88 | 122 | 3,8 |
| 20,564 | 4,32 | 1198 | 37,7 |
| 21,285 | 4,17 | 330 | 10,4 |
| 21,781 | 4,08 | 317 | 10 |
| 22,632 | 3,93 | 190 | 6 |
| 23,308 | 3,81 | 133 | 4,2 |
| 23,797 | 3,74 | 427 | 13,4 |
| 24,276 | 3,66 | 118 | 3,7 |
| 25,190 | 3,53 | 92 | 2,9 |
| 25,924 | 3,43 | 251 | 7,9 |
| 26,646 | 3,34 | 250 | 7,9 |
| 27,620 | 3,23 | 96 | 3 |
| 28,306 | 3,15 | 133 | 4,2 |

2. Composition pharmaceutique contenant comme principe actif le composé selon la revendication 1, en combinaison avec un ou plusieurs véhicules inertes, non toxiques et pharmaceutiquement acceptables.

3. Utilisation du composé selon la revendication 1 pour la fabrication de médicaments utiles en tant qu'inhibiteur de l'enzyme de conversion de l'angiotensine I.

4. Utilisation du composé selon la revendication 1 pour la fabrication de médicaments utiles dans le traitement des maladies cardiovasculaires.

5. Composition pharmaceutique selon la revendication 2 **caractérisée en ce qu'**elle contient également un diurétique.

6. Composition pharmaceutique selon la revendication 5 **caractérisée en ce que** le diurétique est l'indapamide.
